# EUROPEAN PATENT SPECIFICATION

(11) **EP 4 034 127 B1**
(45) Date of publication and mention of the grant of the patent: **04.11.2026**
(21) Application number: 20869906.6
(22) Date of filing: 05.08.2020
(51) Int. Cl.: A61K 9/20, A61K 9/46, A61K 31/519, A61P 3/00

(54) **EFFERVESCENT FORMULATIONS OF SAPROPTERIN DIHYDROCHLORIDE**
BRAUSEFORMULIERUNGEN VON SADPROPTERIN-DIHYDROCHLORID
FORMULATIONS EFFERVESCENTES DE DICHLORHYDRATE DE SAPROPTÉRINE

(30) Priority: 23.09.2019 TR 201914416
(43) Date of publication of application: 03.08.2022
(73) Proprietor: Sanovel Ilac Sanayi ve Ticaret A.S., Sisli - Istanbul (TR)
(72) Inventor: PALANTOKEN, Arzu, 34460 Sariyer/Istanbul (TR); TURKOGLU, Damla, 34460 Sariyer/Istanbul (TR)
(74) Representative: Genç Ilhan, Oznur
(86) International application number: PCT/TR2020/050689
(87) International publication number: WO 2021/061066

(56) References cited:
- EP-A1- 1 757 293
- EP-A1- 3 138 566
- EP-A2- 2 139 485
- WO-A1-2012/127431
- CN-A- 104 257 623
- US-A1- 2010 239 667

## Description

### Field of the invention

The present invention relates to effervescent tablet formulations comprising sapropterin dihydrochloride and one or more pharmaceutically acceptable excipients.

### Background of the invention

Phenylketonuria (PKU) is a rare condition where babies are born unable to break down an amino acid called phenylalanine. This causes phenylalanine to build up. When phenylalanine levels get too high, it can cause damage to the brain. This can lead to intellectual and developmental disabilities.

Phenylalanine is found in foods that contain protein. PKU is manageable, mostly through your diet. The key to living with PKU successfully is finding it early. That is why all babies in the United States are screened for the disease at birth.

PKU is an inherited disease. This means it is passed down through the genes of the mother and father. It is caused by mutations in the gene that helps make an enzyme called phenylalanine hydroxylase. This enzyme helps break down phenylalanine. When this gene doesn't work right, the body can't break down phenylalanine. It starts to build up and causes damage to nerve cells in the brain.

Initially in newborns there are no symptoms. But within a few months of birth, depending on the severity of the disease, symptoms begin to show. These include:
- Smaller than normal head size (called microcephaly)
- Hyperactivity
- A musty or mouse-like odor in urine, breath, or skin
- Lighter skin, hair, and eyes than their siblings
- Skin disorders such as eczema
- Jerking movements of the arms or legs
- Tremors
- Seizures
- Delayed development
- Behavioral problems
- Psychiatric disorders
- Permanent intellectual disability

If PKU is not controlled through changes in diet, it can cause severe intellectual and developmental disability.

The U.S. Food and Drug Administration has approved the medicine sapropterin dihydrochloride (Kuvan) for the treatment of PKU. Kuvan can help the body break down phenylalanine.

Sapropterin dihydrochloride (Kuvan^{®} [BioMarin, CA, USA]) is the first and only registered synthetic form of the naturally occurring enzyme cofactor, BH4 (5,6,7,8-tetrahydrobiopterin) Sapropterin is an orally active, synthetic dihydrochloride salt formulation of the Biologically active 6R-diastereoisomer of BH4 (5,6,7,8-tetrahydrobiopterin) Although PKU is characterized by a defect in the PAH (Phenylalanine hydroxylase) enzyme, residual enzymatic activity may be present in some patients. Thus, sapropterin may act like a chemical chaperone to promote the normal metabolism of Phe (Phenylalanine) and lower its concentration in the blood in a subset of patients who are BH4 responsive. Sapropterin may be used to assist in the control of Phe concentrations. It provides the opportunity for patients who respond to BH4 to adjust their diet, thereby allowing a greater intake of Phe or even coming off of their Phe-restrictive diet. The overall frequency of BH4 responsiveness across Europe is estimated to be 55-62%, based on projections made using genetic allelic data for BH4 responsiveness, although responsiveness can only be determined by a response test.

The chemical name of sapropterin dihydrochloride is (6R)-2-amino-6-[(1R,2S)-1,2-dihydroxypropyl]-5,6,7,8-tetrahydro-4(1H)-pteridinone dihydrochloride and the molecular formula is C9H15N5O3·2HCl with a molecular weight of 314.17. Sapropterin dihydrochloride has the following structural formula:

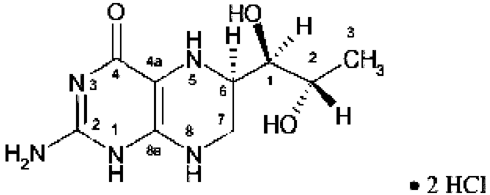

The patent US7727987B2 of Merck discloses crystal forms of (6R)-L-erythro-tetrahydrobiopterin dihydrochloride and hydrates and solvates thereof. Also relates to compositions comprising selected and stable crystal forms of (6R)-L-erythro-tetrahydrobiopterin dihydrochloride or a hydrate thereof and a pharmaceutically acceptable carrier.

The patent EP1757293B1 of Daiichi Sankyo discloses an effective sapropterin hydrochloride preparation that is highly stable during storage and can administer sapropterin hydrochloride, an effective therapeutic agent for the treatment of tetrahydrobiopterin-responsive hyperphenylalaninemia, to patients of a wide range of ages, ranging from infants to adults, in a single preparation.

The patent application EP2139485 of BioMarin Pharmaceutical discloses composition and methods for treating BH-4 responsive disorders. BH4 is stabilized in patient's intestinal tract by decreasing intestinal pH, e.g. using proton exchange polymers. Gaz generating flating capsules comprising CH4 and acidifying excipients, such as proton exchange polymers, are also disclosed in this document.

The patent application EP1845952A2 of BioMarin Pharmaceutical discloses a stable solid formulation of tetrahydrobiopterin, or a precursor or derivative or analog thereof, that maintains its stability for an extended period of time. Compositions of the invention may comprise a stable, crystalline form of BH4 that is stable at room temperature for more than 8 hours and a pharmaceutically acceptable carrier, diluent or excipient. Exemplary stable tablets of the invention have been prepared using a dry tableting process and have been shown to have a shelf-life of at least 6 to 9 months at room temperature.

The patent application EP2680848A1 of Dipharma discloses stable pharmaceutical compositions comprising tetrahydrobiopterin and at least one stabilizing agent. Particularly the present invention relates to stable compositions of sapropterin dihydrochloride. Antioxidants that stabilize tetrahydrobiopterin; with the weight ratio of the antioxidant to active ranging from 0.2 - 1 .5.

In prior art, CN104257623A patent of Guangdong Zhongsheng pharmaceutical company discloses an effervescent tablet containing sapropterin dihydrochloride.

Sapropterin dihydrochloride exhibits polymorphism and many crystalline forms have been identified; among all the polymorphic forms, Form B was identified to be thermodynamically stable crystalline anhydrate form. Polymorph form B is a very stable crystalline form, that can be easily filtered off, dried and ground to particle sizes desired for pharmaceutical formulations. These outstanding properties renders polymorph form B especially feasible for pharmaceutical application.

Sapropterin dihydrochloride is currently available as oral soluble tablets of 100 mg and 100 mg-500 mg powder packets under the brand name Kuvan^{™}. It is marketed by BioMarin in the US and Merck Serono in Europe. Kuvan^{™} has been designated as an orphan medication since hyperphenylalaninemia is a rare disease. Kuvan^{™} is indicated to reduce blood phenylalanine levels in patients with hyperphenylalaninemia due to tetrahydrobiopterin responsive phenylketonuria. It is to be used in conjunction with phenylalanine restricted diet. In patients with phenylketonuria the role of sapropterin dihydrochloride is to enable endogenous phenylalanine hydroxylase activity and to partially restore oxidative metabolism of phenylalanine, resulting in decreased blood phenylalanine levels. In patients with BH4 deficiency, sapropterin dihydrochloride is proposed to restore endogenous phenylalanine hydroxylase activity by providing an exogenous source of the missing cofactor.

Effervescent tablets are designed to break down quickly and release carbon dioxide when dropped in liquid. They have gained considerable attention as a preferred alternative to conventional tablets and capsules due to their better patient compliance. Today, there are a growing number of people who cannot swallow tablets or capsules. On the other side, in effervescent formulations the active ingredient is already solubilized before its administration and liquid effervescent form is easier to take as compared to tablets or capsules.

Advantages of effervescent tablets: Improved taste, faster absorpsiton, presentable fizzy tablets.

Taking big tablets or capsules is difficult for the patients. Effervescent technology provides an alternative to them. Dissolving and break-down of standard tablets also takes additional time in the stomach. In effervescents, ingredients are distributed in the solution and they are not localized in certain point. They can be taken in liquids and promotes patients to take more liquid. Absorption is improved and usage is easy in effervescent tablets.

Sapropterin dihydrochloride has a certain pungent odor so unpleasant and bitter taste is problem that should be overcome. Therefore, it is needed to develop an effervescent formulation which has a desired acceptable taste for patients.

The solubility and dissolution rate of sapropterin dihydrochloride directly influence its bioavailability. For this reason, it is quite important to increase the solubility and dissolution rate of sapropterin dihydrochloride. A desired dissolution profile of the effervescent formulation is obtained.

### Detailed Description of the Invention

The present invention provides an effervescent tablet formulation**,** as defined in claim 1, comprising sapropterin dihydrochloride and one or more pharmaceutically acceptable excipients. The term "sapropterin dihydrochloride" as used throughout the specification refers to not only sapropterin dihydrochloride, but also its other pharmaceutically acceptable solvates, pharmaceutically acceptable hydrates, pharmaceutically acceptable enantiomers or pharmaceutically acceptable polymorphs.

According to the invention, sapropterin dihydrochloride is present in an amount of 15.0 to 40.0 %, preferably 20.0 to 35.0 % and more preferably 25.0 to 30.0 % by weight of total formulation.

According to the invention, said one or more pharmaceutically acceptable excipient comprises an acidifying agent and may also comprise an alkalizing agent, lubricants, binders, antioxidants, aromatic agents and sweeteners, diluents, glidants or mixtures thereof.

The effervescent couple includes two mutually reactive components, such as an acid source and a base source, it is preferred that both components react completely. In one embodiment, said effervescent couple is an acid and a base.

According to this embodiment, the acidifying agent is selected from the group consisting of citric acid, fumaric acid, propionic acid, tartaric acid, citric acid monohydrate, citric acid anhydrate, adipic acid, acetic acid, hydrochloric acid, malic acid, nitric acid, phosphoric acid, nicotinic acid, acetyl salicylic acid, sulfuric acid or acid salts (such as amino acid hydrochlorides, sodium citrates, sodium citrate dihydrate, disodium citrate, disodium dihydrogen citrate or sodium acid phosphate) or mixtures thereof.

According to one embodiment of the present invention, the amount of acidifying agent is between 0.5% and 5.0% by weight of the formulation.

According to one embodiment of the present invention, the acidifying agent is citric acid.

According to this embodiment, the alkalizing agent is selected from the group comprising sodium bicarbonate, sodium bisulfite, sodium metabisulfite, sodium carbonate, sodium glycine carbonate, ammonium carbonate, calcium carbonate, potassium bicarbonate, potassium carbonate or magnesium carbonate or mixtures thereof.

According to one embodiment of the present invention, the amount of alkalizing agent is between 25.0% and 50.0% by weight of the formulation, preferably it is between 30.0% and 50.0% by weight, more preferably it is between 40.0% and 50.0% by weight of the formulation.

According to one embodiment of the present invention, the alkalizing agent is sodium bicarbonate.

Another object of the present invention is to provide an effervescent formulation, having a desired level of solubility and dissolution rate, and therefore a desired level of bioavailability, with an effervescent sapropterin dihydrochloride containing formulation.

The ratio of sapropterin dihydrochloride to an acidifiying agent affects dissolution rate of effervescent formulation. This ratio is improving the dissolution profile of the effervescent formulation.

According to the invention, the ratio of sapropterin dihydrochloride to an acidifiying agent is between 4.0 - 70.0(w/w), more preferably 5.0 - 60.0(w/w) in the total formulation.

Suitable antioxidants are selected from the group comprising alpha tocopherol, quercetine, ascorbyl palmitate, butylhydroxyanisole (BHA), alpha lipoic acid, butylhydroxytoluene (BHT), erythorbic acid, monothioglycerol, potassium metabisulfite, propyl gallate, sodium ascorbate, sodium metabisulfite, sodium sulfite, citric acid, thymol or mixtures thereof.

The amount of antioxidant is between 5.6-10.0% preferably 5.6-8.0% and more preferably 5.6-6.4% by weight of the effervescent formulation.

Suitable binders are selected from the group comprising carboxy methyl cellulose , polyvinylpyrrolidone, polyethylene glycol, polyvinyl alcohol, starch, pregelatinized starch, glucose, glucose syrup, natural gums, sucrose, sodium alginate, hydroxypropyl methyl cellulose, hydroxypropyl cellulose, , methyl cellulose, gelatin, carrageenan, guar gum, carbomer, polymethacrylates, methacrylate polymers, collagens, gelatin, agar, alginate, alginic acid, xanthan gum, hyaluronic acid, pectin, polysaccharides, carbomer, poloxamer, polyacrylamide, aluminium hydroxide, laponite, bentonite, polyoxyethylene-alkyl ether, polydextrose, polyethylene oxide or mixtures thereof.

According to one embodiment of the present invention, the amount of binder is between 1.0% and 6.0%, 2.0% and 6.0%, 3.0% and 6.0%, 4.0% and 6.0% by weight of the total formulation.

According to one embodiment of the present invention, the binder is carboxy methyl cellulose.

Suitable lubricants are selected from the group comprising from magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

Suitable aromatic agents are selected from the group comprising fruit aromas such as orange, banana, strawberry, cherry, wild cherry, lemon, or other aromas such as cardamom, anis, mint, menthol, vanillin, or mixtures thereof.

Suitable glidants are selected from the group comprising, colloidal silicon dioxide, corn starch, talc or mixtures thereof.

Suitable sweeteners are selected from the group comprising monoammonium glycyrrhizinate, potassium acesulfame, sodium saccharinate, neohesperidine dihydrochalcone, sucralose, saccharin, sucrose, glucose, lactose, fructose, mannitol, sorbitol, xylitol, erythritol or mixtures thereof.

Suitable diluents which is selected from the group comprising microcrystalline cellulose, lactose monohydrate, lactose, dibasic calcium phosphate, mannitol, spraydried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment of the present invention, the amount of diluent is between 5.0% and 90.0%, 5.0% and 80.0%, 5.0% and 70.0%, 5.0% and 60.0%, 5.0% and 50.0%, 5.0% and 40.0%, 5.0% and 30.0%, 5.0% and 20.0%, 5.0% and 10.0% by weight of total formulation.

According to one embodiment of the present invention, the diluent is microcrystalline cellulose.

According to the present invention, the effervescent formulation is in the form of a tablet.

The effervescent formulation of the present invention is prepared, using standard techniques and manufacturing processes well known in the art, such as direct compression, wet or dry granulation, hot melt granulation, hot melt extrusion, fluidized bed granulation, extrusion, spheronization, slugging, spray drying or solvent evaporation.

### Example 1: Effervescent formulations of sapropterin dihydrochloride processed with dry granulation/direct compression

| **Ingredients** | **Amount (%)** |
|---|---|
| Sapropterin dihydrochloride | 15.0-40.0% |
| Sodium bicarbonate | 25.0-50.0% |
| Citric acid | 0.5-5.0% |
| Carboxymethyl cellulose | 1.0-6.0% |
| Orange flavor | 1.0-2.0% |
| Monoammonium glycyrrhizinate | 5.0-10.0% |
| Microcrystalline cellulose | 5.0-90.0% |
| Antioxidant* | 5.6-10.0% |
| Colloidal silicon dioxide | 0.10-1.0% |
| Magnesium stearate | 0.25-5.0% |
| **TOTAL** | **100** |

### Process: Dry granulation/direct compression

a. Sieving sapropterin dihydrochloride, sodium bicarbonate, citric acid, carboxymethyl cellulose, monoammonium glycyrrhizinate, orange flavor, microcrystalline cellulose, antioxidant* and colloidal silicon dioxide and mixing.
b. Adding magnesium stearate to this mixture and mixing.
c. Compressing this mixture into tablets with low hardness.

* Butylhydroxyanisole, Butylhydroxytoluene, vitamin E, Quercetine or Citric acid can be used as an antioxidant.

### Example 2: Effervescent formulations of sapropterin dihydrochloride processed with dry granulation/direct compression

| **Ingredients** | **Amount (%)** |
|---|---|
| Sapropterin dihydrochloride | 20.0-35.0% |
| Sodium bicarbonate | 30.0-50.0% |
| Citric acid | 0.5-5.0% |
| Carboxy methyl cellulose | 2.0-6.0% |
| Orange flavor | 1.0-2.0% |
| Monoammonium glycyrrhizinate | 5.0-10.0% |
| Microcrystalline cellulose | 5.0-50.0% |
| Antioxidant* | 5.6-8.0 % |
| Colloidal silicon dioxide | 0.10-1.0% |
| Magnesium stearate | 0.25-5.0% |
| **TOTAL** | **100** |

### Process: Dry granulation/direct compression

a. Sieving sapropterin dihydrochloride, sodium bicarbonate, citric acid, carboxymethyl cellulose, monoammonium glycyrrhizinate, orange flavor, microcrystalline cellulose, antioxidant* and colloidal silicon dioxide and mixing.
b. Adding magnesium stearate to this mixture and mixing.
c. Compressing this mixture into tablets with low hardness.

* Butylhydroxyanisole, Butylhydroxytoluene, vitamin E, Quercetine or Citric acid can be used as an antioxidant.

### Example 3: Effervescent formulations of sapropterin dihydrochloride processed with dry granulation/direct compression

| **Ingredients** | **Amount (%)** |
|---|---|
| Sapropterin dihydrochloride | 25.0-30.0% |
| Sodium bicarbonate | 40.0-50.0% |
| Citric acid | 0.5-5.0% |
| Carboxy methyl cellulose | 4.0-6.0% |
| Orange flavor | 1.0-2.0% |
| Monoammonium glycyrrhizinate | 5.0-10.0% |
| Microcrystalline cellulose | 5.0-10.0% |
| Antioxidant* | 5.6-6.4% |
| Colloidal silicon dioxide | 0.10-1.0% |
| Magnesium stearate | 0.25-5.0% |
| **TOTAL** | **100** |

### Process: Dry granulation/direct compression

a. Sieving sapropterin dihydrochloride, sodium bicarbonate, citric acid, carboxymethyl cellulose, monoammonium glycyrrhizinate, orange flavor, microcrystalline cellulose, antioxidant* and colloidal silicon dioxide and mixing.
b. Adding magnesium stearate to this mixture and mixing.
c. Compressing this mixture into tablets with low hardness.

* Butylhydroxyanisole, butylhydroxytoluene, vitamin E, quercetine or citric acid can be used as an antioxidant.

### Example 4: Effervescent formulations of sapropterin dihydrochloride processed with dry granulation/direct compression

| **Ingredients** | **Amount (%)** |
|---|---|
| Sapropterin dihydrochloride | 25.0% |
| Sodium bicarbonate | 45.0% |
| Citric acid | 2.0% |
| Carboxy methyl cellulose | 5.0% |
| Orange flavor | 1.0% |
| Monoammonium glycyrrhizinate | 5.0% |
| Microcrystalline cellulose | 7.5% |
| Antioxidant* | 6.0% |
| Colloidal silicon dioxide | 1.0% |
| Magnesium stearate | 2.5% |
| **TOTAL** | **100** |

### Process: Dry granulation/direct compression

a. Sieving sapropterin dihydrochloride, sodium bicarbonate, citric acid carboxymethyl cellulose, monoammonium glycyrrhizinate, orange flavor, microcrystalline cellulose, antioxidant* and colloidal silicon dioxide and mixing.
b. Adding magnesium stearate to this mixture and mixing.
c. Compressing this mixture into tablets with low hardness.

* Butylhydroxyanisole, butylhydroxytoluene, vitamin E, quercetine or citric acid can be used as an antioxidant.
With this invention, an effervescent formulation comprising sapropterin dihydrochloride is achieved which is eliminating stability, process and disintegration related problems and bringing additional advantages.

## Claims

1. An effervescent tablet formulation comprising sapropterin dihydrochloride or its pharmaceutically acceptable solvates, hydrates, enantiomers or polymorphs and an acidifying agent wherein sapropterin dihydrochloride is present in an amount of 15.0 to 40.0% by weight of total formulation **characterized in that** weight ratio of sapropterin dihydrochloride to acidifying agent is between 4.0 - 70.0 (w/w), preferably between 5.0 - 60.0 (w/w), in total formulation.

2. The effervescent tablet formulation according to claim 1, wherein sapropterin dihydrochloride is in an amount of 20.0 to 35.0 %, preferably 25.0 to 30.0 % by weight of total formulation.

3. The effervescent tablet formulation according to claim 1, wherein the acidifying agent is selected from the group consisting of fumaric acid, propionic acid, tartaric acid, citric acid, citric acid monohydrate, citric acid anhydrate, adipic acid, acetic acid, hydrochloric acid, malic acid, nitric acid, phosphoric acid, nicotinic acid, acetyl salicylic acid, sulfuric acid or acid salts (such as amino acid hydrochlorides, sodium citrates, sodium citrate dihydrate, disodium citrate, disodium dihydrogen citrate or sodium acid phosphate) or mixtures thereof, preferably it is citric acid.

4. The effervescent tablet formulation according to any preceding claim, comprising;
a) 25.0 - 30.0 % by weight of sapropterin dihydrochloride
b) 40.0 - 50.0 % by weight of sodium bicarbonate
c) 0.5 - 5.0 % by weight of citric acid
d) 4.0 - 6.0 % by weight of carboxy methyl cellulose
e) 1.0 - 2.0 % by weight of orange flavor
f) 5.0 - 10.0 % by weight of monoammonium glycyrrhizinate
g) 5.0 - 10.0 % by weight of microcrystalline cellulose
h) 5.6 - 6.4 % by weight of quercetine, butyl hydroxyl anisole, or butyl hydroxyl toluene
i) 0.1 - 1.0 % by weight of colloidal silicon dioxide
j) 0.25 - 5.0 % by weight of magnesium stearate

5. The effervescent tablet formulation according to any preceding claims, wherein the effervescent formulation is prepared by using direct compression, wet or dry granulation, hot melt granulation, hot melt extrusion, fluidized bed granulation, extrusion, spheronization, slugging, spray drying or solvent evaporation

## Patentansprüche

1. Eine Brausetablettenformulierung, umfassend Sapropterindihydrochlorid oder dessen pharmazeutisch verträgliche Solvate, Hydrate, Enantiomere oder Polymorphe und ein Säuerungsmittel, wobei Sapropterindihydrochlorid in einer Menge von 15,0 bis 40,0 Gew.-% der Gesamtrezeptur vorhanden ist, **dadurch gekennzeichnet, dass** das Gewichtsverhältnis von Sapropterindihydrochlorid zu Säuerungsmittel in der Gesamtrezeptur zwischen 4,0 und 70,0 (w/w), vorzugsweise zwischen 5,0 und 60,0 (w/w) liegt.

2. Die Brausetablettenformulierung nach Anspruch 1, wobei Sapropterindihydrochlorid in einer Menge von 20,0 bis 35,0 Gew.-%, vorzugsweise 25,0 bis 30,0 Gew.-% der Gesamtrezeptur vorliegt.

3. Die Brausetablettenformulierung nach Anspruch 1, wobei das Säuerungsmittel ausgewählt ist aus der Gruppe bestehend aus Fumarsäure, Propionsäure, Weinsäure, Citronensäure, Citronensäure-Monohydrat, wasserfreier Citronensäure, Adipinsäure, Essigsäure, Salzsäure, Äpfelsäure, Salpetersäure, Phosphorsäure, Nicotinsäure, Acetylsalicylsäure, Schwefelsäure oder sauren Salzen (wie Aminosäure-Hydrochloriden, Natriumcitraten, Natriumcitrat-Dihydrat, Dinatriumcitrat, Dinatriumdihydrogencitrat oder Natriumsäurenphosphat) oder Mischungen davon, wobei es vorzugsweise Citronensäure ist.

4. Die Brausetablettenformulierung nach einem der vorhergehenden Ansprüche, umfassend:
a) 25,0-30,0 Gew.-% Sapropterindihydrochlorid
b) 40,0-50,0 Gew.-% Natriumbicarbonat
c) 0,5-5,0 Gew.-% Citronensäure
d) 4,0-6,0 Gew.-% Carboxymethylcellulose
e) 1,0-2,0 Gew.-% Orangenaroma
f) 5,0-10,0 Gew.-% Monoammoniumglycyrrhizinat
g) 5,0-10,0 Gew.-% mikrokristalline Cellulose
h) 5,6-6,4 Gew.-% Quercetin, Butylhydroxyanisol oder Butylhydroxytoluol
i) 0,1-1,0 Gew.-% collodiales Siliciumdioxid
j) 0,25-5,0 Gew.-% Magnesiumstearat

5. Die Brausetablettenformulierung nach einem der vorhergehenden Ansprüche, wobei die Brauseformulierung unter Verwendung von Direktverpressung, Nass- oder Trockengranulierung, Schmelzgranulierung, Heißschmelzextrusion, Wirbelschichtgranulierung, Extrusion, Sphäronisation, Tablettierung durch Kompaktieren, Sprühtrocknung oder Lösemittelverdampfung hergestellt wird.

## Revendications

1. Formulation de comprimé effervescent comprenant du dichlorhydrate de saproptérine ou ses solvates, hydrates, énantiomères ou polymorphes pharmaceutiquement acceptables et un agent acidifiant, dans laquelle le dichlorhydrate de saproptérine est présent en une quantité de 15,0 à 40,0 % en poids de la formulation totale, **caractérisée en ce que** le rapport pondéral du dichlorhydrate de saproptérine à l'agent acidifiant est compris entre 4,0 - 70,0 (p/p), de préférence entre 5,0 - 60,0 (p/p), dans la formulation totale.

2. Formulation de comprimé effervescent selon la revendication 1, dans laquelle le dichlorhydrate de saproptérine est présent en une quantité de 20,0 à 35,0 %, de préférence de 25,0 à 30,0 % en poids de la formulation totale.

3. Formulation de comprimé effervescent selon la revendication 1, dans laquelle l'agent acidifiant est choisi dans le groupe constitué par l'acide fumarique, l'acide propionique, l'acide tartrique, l'acide citrique, l'acide citrique monohydraté, l'acide citrique anhydre, l'acide adipique, l'acide acétique, l'acide chlorhydrique, l'acide malique, l'acide nitrique, l'acide phosphorique, l'acide nicotinique, l'acide acétylsalicylique, l'acide sulfurique ou des sels d'acides (tels que des chlorhydrates d'acides aminés, des citrates de sodium, le citrate de sodium dihydraté, le citrate disodique, le dihydrogénocitrate disodique ou le phosphate acide de sodium) ou leurs mélanges, de préférence il s'agit de l'acide citrique.

4. Formulation de comprimé effervescent selon l'une quelconque des revendications précédentes, comprenant ;
a) 25,0-30,0 % en poids de dichlorhydrate de saproptérine
b) 40,0-50,0 % en poids de bicarbonate de sodium
c) 0,5-5,0 % en poids d'acide citrique
d) 4,0-6,0 % en poids de carboxyméthylcellulose
e) 1,0-2,0 % en poids d'arôme d'orange
f) 5,0-10,0 % en poids de glycyrrhizinate de monoammonium
g) 5,0-10,0 % en poids de cellulose microcristalline
h) 5,6-6,4 % en poids de quercétine, de butylhydroxyanisole ou de butylhydroxytoluène
i) 0,1-1,0 % en poids de dioxyde de silicium colloïdal
j) 0,25 - 5,0 % en poids de stéarate de magnésium

5. Formulation de comprimé effervescent selon l'une quelconque des revendications précédentes, dans laquelle la formulation effervescente est préparée en utilisant la compression directe, la granulation par voie humide ou sèche, la granulation par fusion, l'extrusion par fusion, la granulation en lit fluidisé, l'extrusion, la sphéronisation, le briquetage (slugging), le séchage par atomisation ou l'évaporation de solvant.
